# EUROPEAN PATENT SPECIFICATION

(11) **EP 3 847 149 B1**
(45) Date of publication and mention of the grant of the patent: **20.04.2022**
(21) Application number: 19755882.8
(22) Date of filing: 15.08.2019
(51) Int. Cl.: C07C 37/20, C07C 39/17

(54) **PROCESS FOR THE PREPARATION OF 3,3,5-TRIMETHYLCYCLOHEXYLIDENE BISPHENOL**
VERFAHREN ZUR HERSTELLUNG VON 3,3,5-TRIMETHYLCYCLOHEXYLIDEN-BISPHENOL
PROCÉDÉ DE PRÉPARATION DE 3,3,5-TRIMÉTHYLCYCLOHEXYLIDÈNE BISPHÉNOL

(30) Priority: 03.09.2018 EP 18192240
(43) Date of publication of application: 14.07.2021
(73) Proprietor: Covestro Intellectual Property GmbH & Co. KG, 51373 Leverkusen (DE)
(72) Inventor: VANDEN EYNDE, Johan, 9052 Zwijnaarde (BE); SLUYTS, Erik, 2930 Brasschaat (BE); HEYLEN, Kristof, 1980 Zemst (BE); TRAVING, Michael, 51399 Burscheid (DE); MICHELE, Volker, 51065 Köln (DE)
(74) Representative: Levpat
(86) International application number: PCT/EP2019/071894
(87) International publication number: WO 2020/048751

(56) References cited:
- EP-A1- 0 995 737
- EP-A1- 1 318 132
- EP-A1- 1 318 135
- EP-B1- 0 995 737
- EP-B1- 1 318 132
- EP-B1- 1 318 135
- GB-A- 1 467 628
- US-A- 2 775 620
- US-A- 3 164 640
- US-A- 4 982 014
- US-A1- 2003 050 514
- US-A1- 2005 165 258
- BAL'TSER A E ET AL: "Effect of Processing Parameters on the Synthesis of Bisphenols, Monomers for Producing Polyester Polymers and Fibres", FIBRE CHEMISTRY, SPRINGER, NEW YORK, NY, US, vol. 47, no. 1, 26 July 2015 (2015-07-26), pages 8-13, XP035528522, ISSN: 0015-0541, DOI: 10.1007/S10692-015-9630-0 [retrieved on 2015-07-26]

## Description

The present inventions relates to the preparation of 3,3,5-trimethylcyclohexylidene bisphenol. Especially, the present invention relates to the preparation of 3,3,5-trimethylcyclohexylidene bisphenol from 3,3,5-trimethylcyclohexanone and phenol in the presence of a gaseous acidic catalyst. The preparation is preferably conducted continuously.

Bisphenols are raw materials for the production of polycondensation materials such as epoxy molding compounds, polyether sulphones, polyether ketones or polycarbonates. Bisphenols are generally produced by reacting phenol or substituted derivatives thereof with suitable ketones in the presence of acidic catalyst and with separation of water. The industrially most significant bisphenol is bisphenol A (BPA), produced from phenol and acetone. Bisphenols derived from cyclic alkanes, for example the condensation product of phenol and 3,3,5-trimethylcylohexanone are also very important in the production of polycarbonates.

The preparation of 3,3,5-trimethylcyclohexylidene bisphenol, hereinafter also referred to as BP-TMC, from 3,3,5-trimethylcyclohexanone, hereinafter referred to as TMC-one, as a first reactant and phenol as a second reactant in a reaction vessel in the presence of a gaseous acidic catalyst is known per se.

Basically the reaction proceeds as follows:

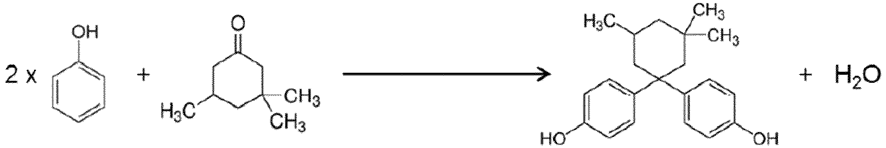

EP0995737A1 discloses the preparation of BP-TMC from TMC-one and phenol in the presence of acidic catalyst already. The acidic catalyst may be a mixture of gaseous hydrogen chloride and an alkylmercaptane for example. EP0995737A1 deals with the problem that BP-TMC causes the viscosity of the reaction mixture to be abnormally high when the reaction between BP-TMC and phenol is proceeded. EP0995737A1 proposes to initially allow phenol and TMC-one to react in a prereaction until at least 90 mol % of the ketone has reacted and then to add a further quantity of phenol and/or aromatic hydrocarbon to the reaction mixture.

EP1277723A1 discloses the preparation of BP-TMC from TMC-one and phenol in the presence of acidic catalyst already, too. The acidic catalyst may be a mixture of gaseous hydrogen chloride and hydrogen sulfide for example. EP1277723A1 is directed to the problem that during the preparation of BP-TMC the reaction mixture becomes solid at a high content of BP-TMC because of the crystallization of BP-TMC.

EP1277723A1 explains that this problem also occurs in the preparation of BP-TMC according to EP0995737A1 but the solution which EP0995737A1 proposes is awkward, does not solve the problem of separating the catalyst and may even introduce a further material into the method which later has to be separated. Therefore, EP1277723A1 proposes to react phenol with TMC-one in the presence of hydrogen chloride and a volatile sulphur compound having an SH bond and separating the resulting bisphenol from unreacted starting materials and catalysts by distillation. The addition of hydrogen chloride and a volatile sulphur compound having an SH bond preferably takes place gaseously by blowing into the reaction mixture. In the process according to EP1277723A1 there is no neutralization and the reaction rate may be slowed or the reaction altogether stopped by adding water. Any volatile components such as catalyst, co-catalyst, water and unreacted raw materials having suitably high volatility may be separated from the reaction mixture by distillation.

However, even the process according to EP1277723A1 does not prevent that an outlet of the dosing valve for the gaseous acidic catalyst is blocked by solids, especially by crystallized BP-TMC, more especially by crystallized BP-TMC-phenol-adduct, when the gas flow is dosed directly into the reaction mixture comprising TMC-one and phenol in a reaction vessel. After a time period, which depends on the dimension of the production apparatuses and the amount or flow rates respectively of the reactants for the preparation of the BP-TMC, the pressure in the dosing valve increases up to a point where it is no longer possible to flow enough catalyst into the reaction vessel to keep a catalyst concentration which is necessary to promote the reaction between TMC-one and phenol. In ordinary industrial processes for the production of BP-TMC such a time period may be 4 to 6 hours e.g. or a few days at the best. After this time period, the dosing valve has to be cleaned which leads to a production hold-up and therefore is expensive. Alternatively, more than one dosing valve has to be provided for dosing the reaction mixture which are operated one after the other and a first dosing valve is cleaned while a second dosing valve is operated. However, even this alternative is inconvenient, causes leakages and impurities, and leads to higher costs. Further alternatively, more than one production unit has to be provided wherein these production units are operated staggered chronologically. While these overcomes the problem of leakage, it is even more expensive because of the multiple units.

Thus, it is an object of the present invention, to overcome the disadvantages of the state of the art. Especially, it is an object of the present invention to prevent that solids, especially crystallized BP-TMC, more especially crystallized BP-TMC-phenol-adduct, block the outlet of the dosing valve for the gaseous acidic acid when the gaseous acidic acid is dosed into the reaction mixture comprising TMC-one and phenol in a reaction vessel.

Surprisingly, the object of the invention is achieved by the subject matter of claim 1. Preferred embodiments are described in the subsequent claims.

Especially the object of the invention is achieved by a process for the preparation of BP-TMC in the presence of a gaseous acidic catalyst, wherein the gaseous acidic catalyst comprises hydrogen sulfide and hydrogen chloride, comprising at least the following steps:
Embodiment (A)
   (a1) providing a separate first stream comprising:
      (i) 3,3,5-trimethylcyclohexanone (TMC-one), the TMC-one having a purity of at least 90 wt.-%, preferably of at least 95 wt.-%, more preferably of at least 98 wt.-%, and the TMC-one comprising less than 1 wt.-%, preferably less than 0,5 wt.-%, more preferably less than 0.2 wt.-%, most preferably less than 0.1 wt.-%, phenol,
      (ii) a gaseous acidic catalyst;
   (b1) providing a separate second stream comprising:
      (iii) phenol,
      (iv) further components;
      and then
   (c) bringing together the first stream and the second stream in a reaction vessel to form a reaction mixture.
Embodiment (B) Alternatively especially the object of the invention is achieved by a process for the preparation of BP-TMC in the presence of a gaseous acidic catalyst, wherein the gaseous acidic catalyst comprises hydrogen sulfide and hydrogen chloride, comprising at least the following steps:
   (a2) providing a separate first stream comprising:
      (v) phenol, the phenol having a purity of at least 90 wt.-%, preferably of at least 95 wt.-%, more preferably of at least 99 wt.-%, most preferably of at least 99.9 wt.-%, and the phenol comprising less than 0.5 wt.-%, preferably less than 0.2 wt.-%, more preferably less than 0.1 wt.-%, most preferably less than 0.05 wt.-% TMC-one,
      (vi) a gaseous acidic catalyst;
   (b2) providing a separate second stream comprising:
      (vii) phenol,
      (viii) TMC-one,
      (ix) further components; and then
   (c) bringing together the first stream and the second stream in a reaction vessel to form a reaction mixture.

So, in other words, the object of the invention especially is achieved by a process for the preparation of BP-TMC in the presence of a gaseous acidic catalyst, wherein the gaseous acidic catalyst comprises hydrogen sulfide and hydrogen chloride, comprising at least the following steps: either
(a1) providing a separate first stream comprising:
   (i) 3,3,5-trimethylcyclohexanone (TMC-one), the TMC-one having a purity of at least 90 wt.-%, preferably of at least 95 wt.-%, more preferably of at least 98 wt.-%, and the TMC-one comprising less than 1 wt.-%, preferably less than 0,5 wt.-%, more preferably less than 0.2 wt.-%, most preferably less than 0.1 wt.-%, phenol,
   (ii) a gaseous acidic catalyst;
(b1) providing a separate second stream comprising:
   (iii) phenol,
   (iv) further components;
   or
(a2) providing a separate first stream comprising:
   (v) phenol, the phenol having a purity of at least 90 wt.-%, preferably of at least 95 wt.-%, more preferably of at least 99 wt.-%, most preferably of at least 99.9 wt.-%, and less than 0.5 wt.-%, preferably less than 0.2 wt.-%, more preferably less than 0.1 wt.-%, most preferably less than 0.05 wt.-% TMC-one,
   (vi) a gaseous acidic catalyst;
(b2) providing a separate second stream comprising:
   (vii) phenol, ,
   (vii) TMC-one,
   (xi) further components; and then
(c) bringing together the first stream and the second stream in a reaction vessel to form a reaction mixture.

Unless explained to the contrary, all explanation in this description both refer to Embodiment (A) and to Embodiment (B).

Preferably, each of the streams (a1), (b1), (a2), and (b2) are conducted into the reaction vessel by a separate pipe. Each of these pipes ends with an orifice which is below the surface of the reaction mixture.

By means of the process according to the invention it is prevented that an outlet of the dosing valve for the gaseous acidic catalyst is blocked by solids, especially by crystallized BP-TMC, more especially by crystallized BP-TMC-phenol-adduct, when the gas flow is dosed directly into the reaction mixture comprising TMC-one and phenol in a reaction vessel.

To achieve the goal of the invention, it is important that the separate stream which comprises the gaseous acidic catalyst either only comprises a very low amount of TMC-one if the stream comprises a high amount of phenol or only comprises a very low amount of phenol if the stream comprises a high amount of TMC-one.

Preferably, the TMC-one in one or more of steps (a1), (a2), and (b2) is in the liquid state. Further preferably, the phenol in one or more of steps (a1), (b1), (a2), and (b2) is in the liquid state.

Preferably, the process is conducted continuously.

Each of the streams (a1), (b1), (a2), and (b2) are conducted into the reaction vessel at the same time, i.e. simultaneously.

Preferably, in step (a1) in the first separate stream TMC-one is present in an amount of 40 to 80 wt.-%, preferably 50 to 70 wt.-%, more preferably 55 to 65 wt.-%, especially in an amount of about 60 wt.-%, and the gaseous acidic catalyst is present in the first separate stream in an amount of from 20 to 60 wt.-%, preferably in an amount of from 30 to 50 wt.-%, more preferably in an amount of 35 to 45 wt.-%, especially in an amount of about 40 wt.-%, wherein the sum of the amounts of TMC-one and gaseous acidic catalyst is 100 wt.-%.

Preferably, in step (b1) in the second separate stream, phenol is present in an amount of at least 75 wt.-%, preferably in an amount of at least 80 wt.-%, more preferably in an amount of at least 85 wt.-%, TMC-one is present in an amount of less than 6 wt.-%, preferably in an amount of less than 2 wt.-%, more preferably in an amount of less than 1 wt.-%, further components are present in an amount of less than 25 wt.-%, preferably in an amount of less than 20 wt.-%, more preferably in an amount of less than 15 wt.%, wherein the sum of the amounts of phenol and further components is 100 wt.-%.

In one embodiment of the invention, these further components in step (b1) in the second separate stream are only inevitable impurities of the utilized phenol originating from the corresponding raw material. In this case the amount of impurities even is less than 10 wt.-%, preferably less than 5 wt.%, more preferably less than 2 wt.-%, most preferably less than 1 wt.-%. In this embodiment it is desired that the phenol is as pure as possible.

In another embodiment of the invention, these further components in step (b1) in the second separate stream comprise TMC-one, BP-TMC and by-products, e.g. isomers of BP-TMC. There may be inevitable impurities originating from the raw materials, too.

Preferably, in this other embodiment of the invention, in step (b1) in the second separate stream, phenol is present in an amount of at least 75 wt.-%, preferably in an amount of at least 80 wt.-%, more preferably in an amount of at least 85 wt.-%, TMC-one is present in an amount of less than 6 wt.-%, preferably in an amount of less than 2 wt.-%, more preferably in an amount of less than 1 wt.-%, BP-TMC is present in an amount of less than 5 wt.-%, preferably in an amount of less than 3 wt.-%, more preferably in an amount of less than 1 wt.-%, by-products are present in an amount of less than 23 wt.-%, preferably in an amount of less than 15 wt.-%, more preferably in an amount of less than 4 wt.-%, wherein the sum of the amounts of phenol, TMC-one, BP-TMC, and by-products is 100 wt.-%.

Preferably, in step (a2) in the first separate stream phenol is present in an amount of from 40 to 80 wt.-%, preferably in an amount of from 50 to 70 wt.-%, more preferably in an amount of from 55 to 65 wt.-%, especially in an amount of about 60 wt.-%, and the gaseous acidic catalyst is present in an amount of from 20 to 60 wt.-%, preferably in an amount of from 30 to 50 wt.-%, more preferably in an amount of 35 to 45 wt.-%, especially in an amount of about 40 wt.-%, wherein the sum of the amounts of phenol and gaseous acidic catalyst is 100 wt.-%.

Preferably, in step (b2) in the second separate stream, phenol is present in an amount of at least 65 wt.-%, preferably in an amount of at least 70 wt.-%, more preferably in an amount of at least 75 wt.-%, TMC-one is present in an amount of 15 to 25 wt.-%, further components are present in an amount of less than 20 wt.-%, preferably in an amount of less than 15 wt.-%, more preferably in an amount of less than 10 wt.%, wherein the sum of the amounts of phenol and further components is 100 wt.-%.

In one embodiment of the invention, these further components in step (b2) in the second separate stream are only inevitable impurities of the utilized phenol originating from the corresponding raw material or the utilized TCM-one originating from the corresponding raw material. In this case the amount of impurities even is less than 5 wt.-%, preferably less than 2 wt.%, more preferably less than 1 wt.-%. In this embodiment it is desired that both the phenol and the TMC-one are as pure as possible.

In another embodiment of the invention, these further components in step (b2) in the second separate stream comprise BP-TMC and by-products, e.g. isomers of BP-TMC. There may be inevitable impurities originating from the raw materials, too.

Preferably, in this other embodiment of the invention, in step (b2) in the second separate stream, phenol is present in an amount of at least 65 wt.-%, preferably in an amount of at least 70 wt.-%, more preferably in an amount of at least 75 wt.-%, TMC-one is present in an amount of 15 to 25 wt.-%, BP-TMC is present in amount of less than 5 wt.-%, preferably in an amount of less than 3 wt.-%, more preferably in an amount of less than 1 wt.-%, by-products are present in an amount of less than 19 wt.-%, preferably in an amount of less than 12 wt.-%, more preferably in an amount of less than 9 wt.-%, wherein the sum of the amounts of phenol, TMC-one, BP-TMC, and by-products is 100 wt.-%.

Independently from which the embodiment is selected, the following conditions are preferred:
The gaseous acidic catalyst comprises hydrogen chloride and hydrogen sulfide. Preferably, the molar ratio between hydrogen chloride and hydrogen sulfide is from 4 : 1 to 20 : 1, more preferably from 6 : 1 to 15 : 1, most preferably from 8 : 1 to 12 : 1, especially 10 : 1.

The gaseous acidic catalyst which is fed into the first separate stream may be mixed with an inert gas, for example nitrogen.

Preferably, in regard to the stoichiometric amount of the reaction between TMC-one and phenol to form BP-TMC the molar amount of phenol is in excess in comparison to the molar amount of TMC-one.

Preferably, the ratio between the mass flow of the first separate stream and the mass flow of the second separate stream is from 2 : 25 to 15 : 25, preferably from 2 : 10 to 1 : 10.

The content of water in the streams (a1), (b1), (a2), (b2) is lower than 0,2 wt.-%. The origin of the water is mainly from the utilized raw materials phenol and TCM-one, i.e. this water is an inevitable impurity of the raw materials. Due to the fact that water hinders the reaction of TMC-one and phenol towards BP-TMC, the water content should be kept low.

Preferably, the temperature in the reaction vessel is at least 30°C and at most 40 °C, preferably at least 31 °C and at most 35°C, especially 32 °C. Preferably, the pressure in the reaction vessel is at least 1 bar absolute and at most 10 bar absolute, preferably at least 1 bar absolute and at most 5 bar absolute, most preferably at least 1 bar absolute and at most 2 bar absolute.

Preferably, the reaction is conducted under three-phase conditions. This means, that there are solid, liquid and gaseous components in the reaction vessel simultaneously. These components are the reactants TMC-one and phenol, the catalyst, the product BP-TMC, water and by-products. Further, there may be inevitable impurities carried in by the reactants used. The formed BP-TMC is present in the solid state in the form of crystals of a BP-TMC-phenol-adduct mainly, i.e. more than 90 wt.-%, preferably more than 95 wt.-%, of the obtained BP-TMC; a minor part of the formed BP-TMC is dissolved in phenol, i.e. less than 10 wt.-% preferably less than 5 wt.-%, of the obtained BP-TMC.

Preferably, the reaction vessel is a stirred tank reactor or a loop flow reactor, preferably a stirred tank reactor.

Preferably, in a step (d) a product stream comprising from 55 to 70 wt.-% unreacted phenol, less than 5 wt.% unreacted TMC-one, from 15 to 22 wt.-% BP-TMC, and from 3.5 to 5.5 wt.-% dissolved acidic catalyst originated from the gaseous acidic catalyst now being dissolved in this product stream, 0,5 to 2 wt.-%, , preferably about 1 wt.-% water, and either from 5 to 20 wt.-%, preferably from 10 to 15 wt.-% of by-products, or from 1 to 4 wt.-%, preferably from 2 to 3 wt.-% of by-products, wherein the sum of the amounts of unreacted phenol, unreacted TMC-one, BP-TMC, water and by-products is 100 wt.-%, is removed from the reaction vessel.

Preferably, in a step (e) the gaseous catalyst and water are removed from the product stream, preferably by distillation.

Preferably, in a step (f) the BP-TMC-phenol-adduct is separated and removed from the product stream, preferably by filtration. In case that in step (e) the dissolved catalyst and water are removed from the product stream by distillation, the BP-TMC-phenol-adduct is converted to dissolved BP-TMC because of the higher temperature during the distillation. So, to be able to remove it by filtration, the dissolved BP-TMC has to be converted into crystals of a BP-TMC-phenol-adduct again; this is preferably done by lowering the temperature, e.g. in a crystallisation unit.

Preferably, the BP-TMC is obtained from the BP-TMC-phenol-adduct by extraction, drying and/or liquid evaporation, preferably by drying.

In a preferred embodiment, after removal of BP-TMC from the product stream in step (f), the remainder of this product stream is enriched with TMC-one, and returned either to the separate second stream of step (b1) or to the separate second stream of step (b2). This returning back either to the separate second stream of step (b1) or to the separate second stream of step (b2) is regarded as step (g). The water content in the remainder of the product stream before the enrichment with TCM-one is below 10 ppm.

By means of this embodiment, large amounts of phenol are can be recuperated. Preferably, there is no additional cleaning of the remainder of the product stream. This saves effort and minimizes waste material.

For the purpose of the present invention, the remainder of the product stream, which was enriched with TMC-one and possibly enriched with phenol, is regarded as mother liquor. This mother liquor comprises at least 75 wt.-%, preferably at least 80 wt.-%, more preferably at least 85 wt.-% of phenol, less than 5 wt.-% of TMC-one, less than 5 wt.-% of dissolved BP-TMC, and from 10 to 15 wt.% of by-products.

By means of the preferred process according to the invention it is not only prevented that an outlet of the dosing valve for the gaseous acidic catalyst is blocked by solids, especially by crystallized BP-TMC, more especially by crystallized BP-TMC-phenol-adduct, but also large amounts of the introduced components, especially phenol and TMC-one, can be recycled and introduced into the process again.

Not falling within the scope of the claimed subject-matter is a device that is set up for carrying out and/or controlling the process described above, or comprises the respective means for carrying out and/or controlling the steps of the process described above.

Means of the device not covered by the claims may comprise hardware and/or software components. The means may for example comprise at least one memory with program instructions of a computer program and at least one processor designed for executing program instructions from the at least one memory. Accordingly, it is also intended not covered by the claims that a device which comprises at least one processor and at least one memory with program instructions is understood as disclosed, wherein the at least one memory and the program instructions are set up to act together with the at least one processor in making the output module or the sensor module carry out and/or control the process according to the invention.

As an alternative or in addition, the means of the device may also comprise one or more sensors and/or one or more communication interfaces.

A communication interface is intended to be understood as meaning for example a wireless communication interface and/or a wire-bound communication interface.

A wireless communication interface is for example a communication interface according to a wireless communication technology. An example of a wireless communication technology is a local radio network technology such as Radio Frequency Identification (RFID) and/or Near Field Communication (NFC) and/or Bluetooth (for example Bluetooth Version 2.1 and/or 4.0) and/or Wireless Local Area Network (WLAN). RFID and NFC are for example specified according to the ISO standards 18000, 11784/11785 and the ISO/IEC standard 14443-A and 15693. WLAN is for example specified in the standards of the IEEE-802.11 family. Another example of a wireless communication technology is a trans-regional radio network technology, such as for example a mobile radio technology, for example the Global System for Mobile Communications (GSM) and/or Universal Mobile Telecommunications System (UMTS) and/or Long Term Evolution (LTE). The GSM, UMTS und LTE specifications are maintained and developed by the 3rd Generation Partnership Project (3GPP).

A wire-bound communication interface is for example a communication interface according to a wire-bound communication technology. Examples of a wire-bound communication technology are a Local Area Network (LAN) and/or a bus system, for example a Controller Area Network bus (CAN bus) and/or a Universal Serial Bus (USB). A CAN bus is for example specified according to the ISO standard ISO 11898. LAN is for example specified in the standards of the IEEE-802.3 family. It goes without saying that the output module and/or the sensor module may also comprise other means not mentioned here.

Furthermore, a computer program not covered by the claims is disclosed, comprising program instructions which are designed to make a device carry out and/or control the process according to present invention when the computer program is executed by a processor.

Also disclosed is a computer-readable storage medium which contains a computer program not covered by the claims. A computer-readable storage medium may for example be designed as a magnetic, electrical, electromagnetic, optical and/or other storage medium. Such a computer-readable storage medium is preferably physical (that is to say "tangible"), for example is designed as a data carrier device. Such a data carrier device is for example portable or permanently installed in a device. Examples of such a data carrier device are volatile or nonvolatile memories with random access (RAM) such as for example NOR flash memories or with sequential access such as NAND flash memories and/or memories with read-only access (ROM) or read and write access. Computer-readable is intended to be understood for example as meaning that the storage medium can be read from and/or written to by a computer or a server device, for example by a processor.

The term open-loop control is understood in the context of the present disclosure as meaning generally an influencing of one or more production parameters by way of one or more devices. Such open-loop control is included by a closed-loop control in dependence on the result of a comparison between an actual value and a setpoint value, wherein for example a production parameter controlled in an open-loop manner has in turn influence on a further iteration of the production parameter controlled in such a way in a closed-loop manner.

The following figures shall give an example of a possible embodiment of the invention. However, the invention shall not be reduced to this embodiment.

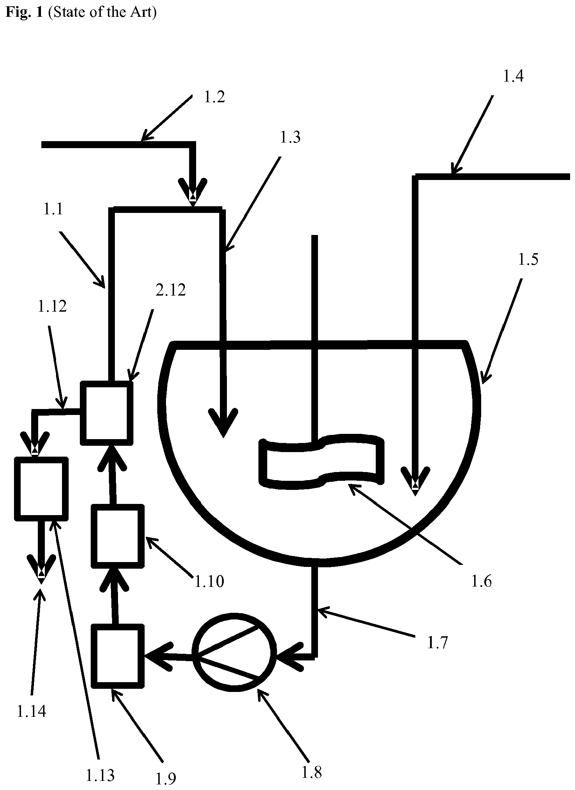

Fig. 1 shows a schematic embodiment of an apparatus according to the state of the art. The reference numbers designate the following items:
- 1.1: a first stream comprising mother liquor,
- 1.2: a second stream comprising freshly added TMC-one and freshly added phenol,
- 1.3: a third stream resulting from the first stream (1.1), the second stream (1.2),

- 1.4: a fourth stream comprising a gaseous acidic catalyst,
- 1.5: reaction vessel,
- 1.6: stirrer,
- 1.7: a fifth stream, being a product stream, comprising unreacted phenol, unreacted TMC-one, BP-TMC (BP-TMC-phenol-adduct and dissolved BP-TMC), dissolved acidic catalyst, water and by-products,
- 1.8: pump,
- 1.9: distillation column,
- 1.10: crystallization unit,
- 1.11: filtration unit,
- 1.12: separated BP-TMC-phenol-adduct,
- 1.13: dryer,
- 1.14: BP-TMC crystals.

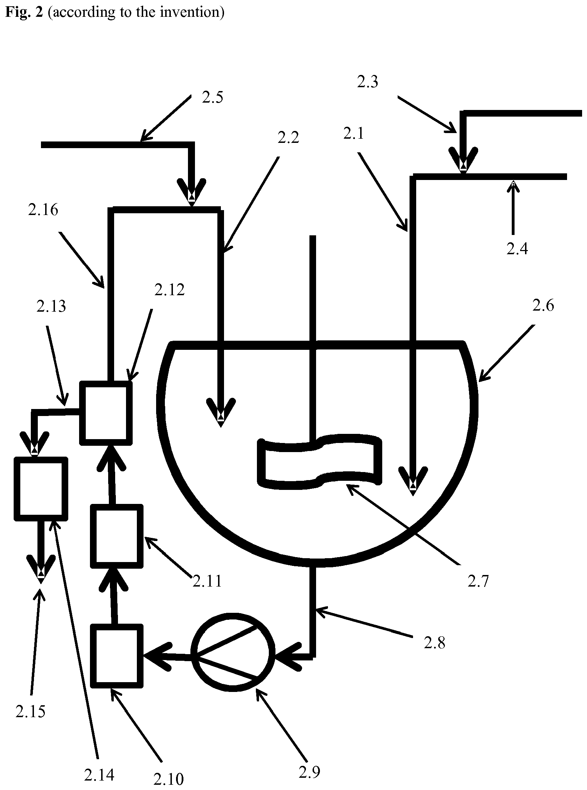

Fig. 2 shows a schematic embodiment of an apparatus used in a process according to the invention. The reference numbers designate the following items:
- 2.1: a first stream comprising phenol of a purity of at least 90 wt.-% and a gaseous acidic catalyst,
- 2.2: a second stream comprising freshly added TMC-one, freshly added phenol, and mother liquor comprising unreacted phenol, unreacted TMC-one, dissolved BP-TMC, and by-products,
- 2.3: a third stream comprising a mixture of a gaseous acidic catalyst,
- 2.4: a fourth stream comprising phenol of a purity of at least 90 wt.-%,
- 2.5: a fifth stream comprising freshly added TMC-one and freshly added phenol,
- 2.6: reaction vessel,
- 2.7: stirrer,
- 2.8: a sixth stream, being the product stream, comprising unreacted phenol, unreacted TMC-one, BP-TMC (both BP-TMC-phenol-adduct and dissolved BP-TMC), dissolved acidic catalyst, water, and by-products, this sixth stream is removed from the reaction vessel (2.6),
- 2.9: pump,
- 2.10: distillation column,
- 2.11: crystallization unit,
- 2.12: filtration unit,
- 2.13: separated BP-TMC-phenol-adduct (crystals),
- 2.14: dryer,
- 2.15: BP-TMC crystals,
- 2.16: mother liquor, comprising unreacted phenol, unreacted TMC-one, dissolved BP-TMC, and by-products, this.

The following description relates to an embodiment according to the invention using an apparatus depicted schematically in Fig. 2. However, the invention shall not be reduced to this embodiment.

A first stream (2.1) comprising phenol and a gaseous acidic catalyst is passed into the reaction vessel (2.6). This first stream (2.1) is formed by introducing a third stream (2.3) comprising a gaseous acidic catalyst into a fourth stream (2.4) comprising phenol.

A second stream (2.2) comprising freshly added TMC-one, freshly added phenol, and mother liquor comprising unreacted phenol, unreacted TMC-one, dissolved BP-TMC, and by-products, is passed into the reaction vessel (2.6), too. This second stream (2.2) is formed by introducing a fifth stream (2.5) comprising freshly added TMC-one and freshly added phenol into an eighth stream (2.11), being a fraction of the sixth stream (2.8).

In the reaction vessel (2.6), which is a stirred tank reactor with a stirrer (2.7), the BP-TMC is formed by a reaction between TMC-one and phenol in the presence of a gaseous acidic catalyst. Besides BP-TMC water is formed by this reaction. By-products such as isomers of BP-TMC e.g. are formed, too.

While TMC-one and phenol are present in the liquid state in the reaction vessel (2.6), the formed BP-TMC is present in the solid state in the form of a BP-TMC-phenol-adduct mainly; a minor part of the formed BP-TMC is dissolved in phenol. As the gaseous acidic catalyst is present in the gaseous state the reaction is conducted under three-phase conditions.

The molar amount of phenol passed into the reaction vessel (2.6) is about 5 to 10, preferably 6 to 7 times the amount which is needed in comparison to the stoichiometric amount of the reaction between TMC-one and phenol to form BP-TMC. This is to ensure that TMC-one reacts to BP-TMC at least nearly quantitatively.

At the bottom of the reaction vessel (2.6) a sixth stream (2.8), being the product stream, comprising unreacted phenol, unreacted TMC-one, BP-TMC (both BP-TMC-phenol-adduct and dissolved BP-TMC), dissolved acidic catalyst and inert gas is removed from the reaction vessel (2.6). This sixth stream (2.8) is conveyed by a pump (2.9).

This sixth stream (2.8) is fed to a distillation unit (2.10), where the dissolved acidic catalyst and water are removed from the sixth stream (2.8).

The sixth stream (2.8) then is fed to a crystallization unit (2.11). In this crystallization unit the dissolved BP-TMC is converted into crystals of a BP-TMC-phenol-adduct.

After that the sixth stream (2.8) is fed to a filtration unit. BP-TMC-phenol-adduct (2.13), preferably in the form of crystals, is obtained from the filtration unit (2.12). These crystals are dried in a dryer (2.14); after that, BP-TMC in the form of crystals is obtained.

The remainder of the sixth stream (2.8) - after removing dissolved acidic catalyst and most of the BP-TMC - forms the mother liquor which is enriched with fresh TMC-one and fresh phenol and is conducted back as seventh stream (2.16), the joined stream forming the second stream (2.2).

## Claims

1. A continuously conducted process for the preparation of 3,3,5-trimethylcyclohexylidene bisphenol (BP-TMC) in the presence of a gaseous acidic catalyst, wherein the gaseous acidic catalyst comprises hydrogen sulfide and hydrogen chloride, comprising at least the following steps:
either
(a1) providing a separate first stream comprising:
(i) 3,3,5-trimethylcyclohexanone (TMC-one), the TMC-one having a purity of at least 90 wt.-%, preferably of at least 95 wt.-%, more preferably of at least 98 wt.-%, and the TMC-one comprising less than 1 wt.-%, preferably less than 0.5 wt.-%, more preferably less than 0.2 wt.-%, most preferably less than 0.1 wt.-%, phenol,
(ii) a gaseous acidic catalyst;
(b1) providing a separate second stream comprising:
(iii) phenol,
(iv) further components;
or
(a2) providing a separate first stream comprising:
(v) phenol, the phenol having a purity of at least 90 wt.-%, preferably of at least 95 wt.-%, more preferably of at least 99 wt.-%, most preferably of at least 99.9 wt.-%, and the phenol comprising less than 0.5 wt.-%, preferably less than 0.2 wt.-%, more preferably less than 0.1 wt.-%, most preferably less than 0.05 wt.-% TMC-one,
(vi) a gaseous acidic catalyst;
(b2) providing a separate second stream comprising:
(vii) phenol,
(viii) TMC-one,
(ix) further components;
and then
(c) bringing together the first stream and the second stream in a reaction vessel to form a reaction mixture.

2. The process of claim 1, wherein in step (a1) in the first separate stream TMC-one is present in an amount of from 40 to 80 wt.-%, preferably in an amount of from 50 to 70 wt.-%, more preferably in an amount of from 55 to 65 wt.-%, and the gaseous acidic catalyst is present in an amount of from 20 to 60 wt.-%, preferably in an amount of from 30 to 50 wt.-%, more preferably in an amount of from 35 to 45 wt.-%, wherein the sum of the amounts of TMC-one and gaseous acidic catalyst is 100 wt.-%.

3. The process of claim 1, wherein in step (b1) in the second separate stream
phenol is present in an amount of at least 75 wt.-%, preferably in an amount of at least 80 wt.-%, more preferably in an amount of at least 85 wt.-%,
further components are present in an amount of less than 25 wt.-%, preferably in an amount of less than 20 wt.-%, more preferably in an amount of less than 15 wt.%, wherein the sum of the amounts of phenol and further components is 100 wt.-%.

4. The process of claim 1, wherein in step (b1) the separate second stream as further components also comprises TMC-one, BP-TMC and by-products.

5. The process of claim 5, wherein in step (b1) in the second separate stream
phenol is present in an amount of at least 75 wt.-%, preferably in an amount of at least 80 wt.-%, more preferably in an amount of at least 85 wt.-%,
TMC-one is present in an amount of less than 6 wt.-%, preferably in an amount of less than 2 wt.-%, more preferably in an amount of less than 1 wt.-%,
BP-TMC is present in an amount of less than 5 wt.-%, preferably in an amount of less than 3 wt.-%, more preferably in an amount of less than 1 wt.-%,
by-products are present in an amount of less than 23 wt.-%, preferably in an amount of less than 15 wt.-%, more preferably in an amount of less than 4 wt.-%,
wherein the sum of the amounts of phenol, TMC-one, BP-TMC, and by-products is 100 wt.-%.

6. The process of claim 1, wherein in step (a2) in the first separate stream
phenol is present in an amount of from 40 to 80 wt.-%, preferably in an amount of from 50 to 70 wt.-%, more preferably in an amount of from 55 to 65 wt.-%, and
the gaseous acidic catalyst is present in an amount of from 20 to 60 wt.-%, preferably in an amount of from 30 to 50 wt.-%, more preferably in an amount of from 35 to 45 wt.-%, wherein the sum of the amounts of phenol and gaseous acidic catalyst is 100 wt.-%.

7. The process of claim 1, wherein in step (b2) in the second separate stream
phenol is present in an amount of at least 65 wt.-%, preferably in an amount of at least 70 wt.-%, more preferably in an amount of at least 75 wt.-%,
TMC-one is present in an amount of 15 to 25 wt.-%,
further components are present in an amount of less than 20 wt.-%, preferably in an amount of less than 15 wt.-%, more preferably in an amount of less than 10 wt.%, wherein the sum of the amounts of phenol, TMC-one and further components is 100 wt.-%.

8. The process of claim 1, wherein in step (b2) the separate second stream as further components also comprises BP-TMC and by-products.

9. The process of claim 9, wherein in step (b2) in the second separate stream
phenol is present in an amount of at least 65 wt.-%, preferably in an amount of at least 70 wt.-%, more preferably in an amount of at least 75 wt.-%,
TMC-one is present in an amount of 15 to 25 wt.-%,
BP-TMC is present in amount of less than 5 wt.-%, preferably in amount of less than 3 wt.-%, more preferably in amount of less than 1 wt.-%,
by-products are present in an amount of less than 19 wt.-%, preferably in amount of less than 12 wt.-%, more preferably in amount of less than 9 wt.-%,
wherein the sum of the amounts of phenol, TMC-one, BP-TMC, and by-products is 100 wt.-%.

10. The process of claim 1, wherein the molar ratio between hydrogen chloride and hydrogen sulfide is from 4 : 1 to 20 : 1, preferably from 6 : 1 to 15 : 1, especially 10 : 1.

11. The process of claim 1, wherein in regard to the stoichiometric amount the total molar amount of phenol in the first separate stream and the second separate stream is in excess in comparison to the total molar amount of TMC-one in the first separate stream and the second separate stream.

12. The process of claim 1, wherein the ratio between the mass rates of the first separate stream and the second separate stream is from 2 : 25 to 15 : 25, preferably from 2 : 10 to 1 : 10.

13. The process of claim 1, wherein in a step (d)
a product stream comprising from 55 to 70 wt.-% unreacted phenol, less than 5 wt.% unreacted TMC-one, from 15 to 22 wt.-% BP-TMC, and 3.5 to 5.5 wt.-% dissolved acidic catalyst, 0,5 to 2 wt.-%, preferably about 1 wt.-% water, and either from 5 to 20 wt.-%, preferably from 10 to 15 wt.-% of by-products, or from 1 to 4 wt.-%, preferably from 2 to 3 wt.-% of by-products, wherein the sum of the amounts of unreacted phenol, unreacted TMC-one, BP-TMC, water and by-products is 100 wt.-%, is removed from the reaction vessel.

## Patentansprüche

1. Kontinuierlich geführtes Verfahren zur Herstellung von 3,3,5-Trimethylcyclohexylidenbisphenol (BP-TMC) in Gegenwart eines gasförmigen sauren Katalysators, wobei der gasförmige saure Katalysator Schwefelwasserstoff und Chlorwasserstoff umfasst, umfassend wenigstens die folgenden Schritte:
Entweder
(a1) Bereitstellen eines gesonderten ersten Stroms, der umfasst:
(i) 3,3,5-Trimethylcyclohexanon (TMC-on), wobei das TMC-on eine Reinheit von wenigstens 90 Gew.-%, vorzugsweise wenigstens 95 Gew.-%, bevorzugter wenigstens 98 Gew.-%, aufweist und das TMC-on weniger als 1 Gew.-%, vorzugsweise weniger als 0,5 Gew.-%, bevorzugter weniger als 0,2 Gew.-%, höchst bevorzugt weniger als 0,1 Gew.-%, Phenol umfasst,
(ii) einen gasförmigen sauren Katalysator;
(b1) Bereitstellen eines gesonderten zweiten Stroms, der umfasst:
(iii) Phenol,
(iv) weitere Komponenten;
oder
(a2) Bereitstellen eines gesonderten ersten Stroms, der umfasst:
(v) Phenol, wobei das Phenol eine Reinheit von wenigstens 90 Gew.-%, vorzugsweise wenigstens 95 Gew.-%, bevorzugter wenigstens 99 Gew.-%, höchst bevorzugt wenigstens 99,9 Gew.-%, aufweist und das Phenol weniger als 0,5 Gew.-%, vorzugsweise weniger als 0,2 Gew.-%, bevorzugter weniger als 0,1 Gew.-%, höchst bevorzugt weniger als 0,05 Gew.-%, TMC-on umfasst,
(vi) einen gasförmigen sauren Katalysator;
(b2) Bereitstellen eines gesonderten zweiten Stroms, der umfasst:
(vii) Phenol,
(viii) TMC-on,
(ix) weitere Komponenten;
und dann
(c) Zusammenbringen des ersten Stroms und des zweiten Stroms in einem Reaktionsgefäß, um ein Reaktionsgemisch zu bilden.

2. Verfahren gemäß Anspruch 1, wobei bei Schritt (a1) in dem ersten gesonderten Strom
TMC-on in einer Menge von 40 bis 80 Gew.-%, vorzugsweise in einer Menge von 50 bis 70 Gew.-%, bevorzugter in einer Menge von 55 bis 65 Gew.-%, vorhanden ist und
der gasförmige saure Katalysator in einer Menge von 20 bis 60 Gew.-%, vorzugsweise in einer Menge von 30 bis 50 Gew.-%, bevorzugter in einer Menge von 35 bis 45 Gew.-%, vorhanden ist,
wobei die Summe der Mengen von TMC-on und gasförmigem saurem Katalysator 100 Gew.-% beträgt.

3. Verfahren gemäß Anspruch 1, wobei bei Schritt (b1) in dem zweiten gesonderten Strom
Phenol in einer Menge von wenigstens 75 Gew.-%, vorzugsweise in einer Menge von wenigstens 80 Gew.-%, bevorzugter in einer Menge von wenigstens 85 Gew.-%, vorhanden ist,
weitere Komponenten in einer Menge von weniger als 25 Gew.-%, vorzugsweise in einer Menge von weniger als 20 Gew.-%, bevorzugter in einer Menge von weniger als 15 Gew.-%, vorhanden sind,
wobei die Summe der Mengen von Phenol und weiteren Komponenten 100 Gew.-% beträgt.

4. Verfahren gemäß Anspruch 1, wobei bei Schritt (b1) der gesonderte zweite Strom als weitere Komponenten auch TMC-on, BP-TMC und Nebenprodukte umfasst.

5. Verfahren gemäß Anspruch 5, wobei bei Schritt (b1) in dem zweiten gesonderten Strom
Phenol in einer Menge von wenigstens 75 Gew.-%, vorzugsweise in einer Menge von wenigstens 80 Gew.-%, bevorzugter in einer Menge von wenigstens 85 Gew.-%, vorhanden ist,
TMC-on in einer Menge von weniger als 6 Gew.-%, vorzugsweise in einer Menge von weniger als 2 Gew.-%, bevorzugter in einer Menge von weniger als 1 Gew.-%, vorhanden ist,
BP-TMC in einer Menge von weniger als 5 Gew.-%, vorzugsweise in einer Menge von weniger als 3 Gew.-%, bevorzugter in einer Menge von weniger als 1 Gew.-%, vorhanden ist,
Nebenprodukte in einer Menge von weniger als 23 Gew.-%, vorzugsweise in einer Menge von weniger als 15 Gew.-%, bevorzugter in einer Menge von weniger als 4 Gew.-%, vorhanden sind,
wobei die Summe der Mengen von Phenol, TMC-on, BP-TMC und Nebenprodukten 100 Gew.-% beträgt.

6. Verfahren gemäß Anspruch 1, wobei bei Schritt (a2) in dem ersten gesonderten Strom
Phenol in einer Menge von 40 bis 80 Gew.-%, vorzugsweise in einer Menge von 50 bis 70 Gew.-%, bevorzugter in einer Menge von 55 bis 65 Gew.-%, vorhanden ist, und
der gasförmige saure Katalysator in einer Menge von 20 bis 60 Gew.-%, vorzugsweise in einer Menge von 30 bis 50 Gew.-%, bevorzugter in einer Menge von 35 bis 45 Gew.-%, vorhanden ist,
wobei die Summe der Mengen von Phenol und gasförmigem saurem Katalysator 100 Gew.-% beträgt.

7. Verfahren gemäß Anspruch 1, wobei bei Schritt (b2) in dem zweiten gesonderten Strom
Phenol in einer Menge von wenigstens 65 Gew.-%, vorzugsweise in einer Menge von wenigstens 70 Gew.-%, bevorzugter in einer Menge von wenigstens 75 Gew.-%, vorhanden ist,
TMC-on in einer Menge von 15 bis 25 Gew.-%, vorhanden ist,
weitere Komponenten in einer Menge von weniger als 20 Gew.-%, vorzugsweise in einer Menge von weniger als 15 Gew.-%, bevorzugter in einer Menge von weniger als 10 Gew.-%, vorhanden sind,
wobei die Summe der Mengen von Phenol, TMC-on und weiteren Komponenten 100 Gew.-% beträgt.

8. Verfahren gemäß Anspruch 1, wobei bei Schritt (b2) der gesonderte zweite Strom als weitere Komponenten ferner BP-TMC und Nebenprodukte umfasst.

9. Verfahren gemäß Anspruch 9, wobei bei Schritt (b2) in dem zweiten gesonderten Strom
Phenol in einer Menge von wenigstens 65 Gew.-%, vorzugsweise in einer Menge von wenigstens 70 Gew.-%, bevorzugter in einer Menge von wenigstens 75 Gew.-%, vorhanden ist,
TMC-on in einer Menge von 15 bis 25 Gew.-%, vorhanden ist,
BP-TMC in einer Menge von weniger als 5 Gew.-%, vorzugsweise in einer Menge von weniger als 3 Gew.-%, bevorzugter in einer Menge von weniger als 1 Gew.-%, vorhanden ist,
Nebenprodukte in einer Menge von weniger als 19 Gew.-%, vorzugsweise in einer Menge von weniger als 12 Gew.-%, bevorzugter in einer Menge von weniger als 9 Gew.-%, vorhanden sind,
wobei die Summe der Mengen von Phenol, TMC-on, BP-TMC und Nebenprodukten 100 Gew.-% beträgt.

10. Verfahren gemäß Anspruch 1, wobei das Molverhältnis zwischen Chlorwasserstoff und Schwefelwasserstoff von 4:1 bis 20:1, vorzugsweise von 6:1 bis 15:1, insbesondere 10:1, beträgt.

11. Verfahren gemäß Anspruch 1, wobei hinsichtlich der stöchiometrischen Menge die Gesamtmolmenge von Phenol in dem ersten gesonderten Strom und dem zweiten gesonderten Strom im Überschuss gegenüber der Gesamtmolmenge von TMC-on in dem ersten gesonderten Strom und dem zweiten gesonderten Strom vorliegt.

12. Verfahren gemäß Anspruch 1, wobei das Verhältnis zwischen den Massenanteilen des ersten gesonderten Stroms und des zweiten gesonderten Stroms von 2:25 bis 15:25, vorzugsweise von 2:10 bis 1:10, beträgt.

13. Verfahren gemäß Anspruch 1, wobei bei einem Schritt (d)
ein Produktstrom, umfassend von 55 bis 70 Gew.-% an unreagiertem Phenol, weniger als 5 Gew.-% an unreagiertem TMC-on, von 15 bis 22 Gew.-% BP-TMC und 3,5 bis 5,5 Gew.-% an ungelöstem saurem Katalysator, 0,5 bis 2 Gew.-%, vorzugsweise etwa 1 Gew.-%, Wasser und entweder von 5 bis 20 Gew.-%, vorzugsweise von 10 bis 15 Gew.-%, an Nebenprodukten oder von 1 bis 4 Gew.-%, vorzugsweise von 2 bis 3 Gew.-%, an Nebenprodukten, wobei die Summe der Mengen von unreagiertem Phenol, unreagiertem TMC-on, BP-TMC, Wasser und Nebenprodukten 100 Gew.-% beträgt, aus dem Reaktionsgefäß entfernt wird.

## Revendications

1. Procédé mis en œuvre en continu pour la préparation de 3,3,5-triméthylcyclohexylidènebisphénol (BP-TMC) en présence d'un catalyseur acide gazeux, dans lequel le catalyseur acide gazeux comprend du sulfure d'hydrogène et du chlorure d'hydrogène, comprenant au moins les étapes suivantes :
soit
(a1) la fourniture d'un premier flux séparé comprenant :
(i) de la 3,3,5-triméthylcyclohexanone (TMC-one), la TMC-one ayant une pureté d'au moins 90 % en poids, de préférence d'au moins 95 % en poids, plus préférablement d'au moins 98 % en poids, et la TMC-one comprenant moins de 1 % en poids, de préférence moins de 0,5 % en poids, plus préférablement moins de 0,2 % en poids, le plus préférablement moins de 0,1 % en poids, de phénol,
(ii) un catalyseur acide gazeux ;
(b1) la fourniture d'un second flux séparé comprenant :
(iii) du phénol,
(iv) d'autres composants ;
soit
(a2) la fourniture d'un premier flux séparé comprenant :
(v) du phénol, le phénol ayant une pureté d'au moins 90 % en poids, de préférence d'au moins 95 % en poids, plus préférablement d'au moins 99 % en poids, le plus préférablement d'au moins 99,9 % en poids, et le phénol comprenant moins de 0,5 % en poids, de préférence moins de 0,2 % en poids, plus préférablement moins de 0,1 % en poids, le plus préférablement moins de 0,05 % en poids de TMC-one,
(vi) un catalyseur acide gazeux ;
(b2) la fourniture d'un second flux séparé comprenant :
(vii) du phénol,
(viii) de la TMC-one,
(ix) d'autres composants ;
puis
(c) la combinaison du premier flux et du second flux dans une cuve de réaction pour former un mélange réactionnel.

2. Procédé selon la revendication 1, dans lequel dans l'étape (a1) dans le premier flux séparé
la TMC-one est présente en une quantité de 40 à 80 % en poids, de préférence en une quantité de 50 à 70 % en poids, plus préférablement en une quantité de 55 à 65 % en poids, et
le catalyseur acide gazeux est présent en une quantité de 20 à 60 % en poids, de préférence en une quantité de 30 à 50 % en poids, plus préférablement en une quantité de 35 à 45 % en poids,
dans lequel la somme des quantités de TMC-one et de catalyseur acide gazeux est de 100 % en poids.

3. Procédé selon la revendication 1, dans lequel dans l'étape (b1) dans le second flux séparé le phénol est présent en une quantité d'au moins 75 % en poids, de préférence en une quantité d'au moins 80 % en poids, plus préférablement en une quantité d'au moins 85 % en poids,
les autres composants sont présents en une quantité de moins de 25 % en poids, de préférence en une quantité de moins de 20 % en poids, plus préférablement en une quantité de moins de 15 % en poids,
dans lequel la somme des quantités de phénol et d'autres composants est de 100 % en poids.

4. Procédé selon la revendication 1, dans lequel dans l'étape (b1) le second flux séparé comprend également en tant qu'autres composants de la TMC-one, du BP-TMC et des sous-produits.

5. Procédé selon la revendication 5, dans lequel dans l'étape (b1) dans le second flux séparé
le phénol est présent en une quantité d'au moins 75 % en poids, de préférence en une quantité d'au moins 80 % en poids, plus préférablement en une quantité d'au moins 85 % en poids,
la TMC-one est présente en une quantité de moins de 6 % en poids, de préférence en une quantité de moins de 2 % en poids, plus préférablement en une quantité de moins de 1 % en poids,
le BP-TMC est présent en une quantité de moins de 5 % en poids, de préférence en une quantité de moins de 3 % en poids, plus préférablement en une quantité de moins de 1 % en poids,
les sous-produits sont présents en une quantité de moins de 23 % en poids, de préférence en une quantité de moins de 15 % en poids, plus préférablement en une quantité de moins de 4 % en poids,
dans lequel la somme des quantités de phénol, de TMC-one, de BP-TMC et de sous-produits est de 100 % en poids.

6. Procédé selon la revendication 1, dans lequel dans l'étape (a2) dans le premier flux séparé
le phénol est présent en une quantité de 40 à 80 % en poids, de préférence en une quantité de 50 à 70 % en poids, plus préférablement en une quantité de 55 à 65 % en poids, et
le catalyseur acide gazeux est présent en une quantité de 20 à 60 % en poids, de préférence en une quantité de 30 à 50 % en poids, plus préférablement en une quantité de 35 à 45 % en poids,
dans lequel la somme des quantités de phénol et de catalyseur acide gazeux est de 100 % en poids.

7. Procédé selon la revendication 1, dans lequel dans l'étape (b2) dans le second flux séparé
le phénol est présent en une quantité d'au moins 65 % en poids, de préférence en une quantité d'au moins 70 % en poids, plus préférablement en une quantité d'au moins 75 % en poids,
la TMC-one est présente en une quantité de 15 à 25 % en poids,
les autres composants sont présents en une quantité de moins de 20 % en poids, de préférence en une quantité de moins de 15 % en poids, plus préférablement en une quantité de moins de 10 % en poids,
dans lequel la somme des quantités de phénol, de TMC-one et d'autres composants est de 100 % en poids.

8. Procédé selon la revendication 1, dans lequel dans l'étape (b2) le second flux séparé comprend également en tant qu'autres composants du BP-TMC et des sous-produits.

9. Procédé selon la revendication 9, dans lequel dans l'étape (b2) dans le second flux séparé
le phénol est présent en une quantité d'au moins 65 % en poids, de préférence en une quantité d'au moins 70 % en poids, plus préférablement en une quantité d'au moins 75 % en poids,
la TMC-one est présente en une quantité de 15 à 25 % en poids,
le BP-TMC est présent en quantité de moins de 5 % en poids, de préférence en quantité de moins de 3 % en poids, plus préférablement en quantité de moins de 1 % en poids,
les sous-produits sont présents en une quantité de moins de 19 % en poids, de préférence en quantité de moins de 12 % en poids, plus préférablement en quantité de moins de 9 % en poids,
dans lequel la somme des quantités de phénol, de TMC-one, de BP-TMC et de sous-produits est de 100 % en poids.

10. Procédé selon la revendication 1, dans lequel le rapport molaire entre le chlorure d'hydrogène et le sulfure d'hydrogène est de 4:1 à 20:1, de préférence de 6:1 à 15:1, en particulier de 10:1.

11. Procédé selon la revendication 1, dans lequel par rapport à la quantité stœchiométrique la quantité molaire totale de phénol dans le premier flux séparé et le second flux séparé est en excès par comparaison avec la quantité molaire totale de TMC-one dans le premier flux séparé et le second flux séparé.

12. Procédé selon la revendication 1, dans lequel le rapport entre les débits massiques du premier flux séparé et du second flux séparé est de 2:25 à 15:25, de préférence de 2:10 à 1:10.

13. Procédé selon la revendication 1, dans lequel dans une étape (d)
un flux de produit comprenant de 55 à 70 % en poids de phénol n'ayant pas réagi, moins de 5 % en poids de TMC-one n'ayant pas réagi, de 15 à 22 % en poids de BP-TMC et 3,5 à 5,5 % en poids de catalyseur acide dissous, 0,5 à 2 % en poids, de préférence environ 1 % en poids d'eau et soit de 5 à 20 % en poids, de préférence de 10 à 15 % en poids de sous-produits, soit de 1 à 4 % en poids, de préférence de 2 à 3 % en poids de sous-produits, dans lequel la somme des quantités de phénol n'ayant pas réagi, de TMC-one n'ayant pas réagi, de BP-TMC, d'eau et de sous-produits est de 100 % en poids, est soutiré de la cuve de réaction.
